# EUROPÄISCHE PATENTSCHRIFT

(11) **EP 3 116 453 B1**
(45) Veröffentlichungstag und Bekanntmachung des Hinweises auf die Patenterteilung: **11.05.2022**
(21) Anmeldenummer: 15717416.0
(22) Anmeldetag: 13.03.2015
(51) Int. Cl.: A61F 2/90, A61F 5/56, D04C 1/06

(54) **KOMPRIMIERBARER SELBSTEXPANDIERBARER STENT ZUR SCHIENUNG UND/ODER OFFENHALTUNG EINES HOHLRAUMS, EINES ORGANWEGS UND/ODER EINES GEFÄSSES IM MENSCHLICHEN ODER TIERISCHEN KÖRPER**
COMPRESSIBLE SELF-EXPANDABLE STENT FOR SPLINTING AND/OR KEEPING OPEN A CAVITY, AN ORGAN DUCT, AND/OR A VESSEL IN THE HUMAN OR ANIMAL BODY
STENT COMPRESSIBLE AUTO-EXPANSIBLE SERVANT À TUTEURER ET/OU MAINTENIR OUVERTE UNE CAVITÉ, UNE VOIE ORGANIQUE ET/OU UN VAISSEAU DE L'ORGANISME HUMAIN OU ANIMAL

(30) Priorität: 13.03.2014 DE 102014003654
(43) Veröffentlichungstag der Anmeldung: 18.01.2017
(73) Patentinhaber: Düring, Klaus, 82494 Krün (DE); Dlaikan-Campos, Nasib, 52146 Würselen (DE)
(72) Erfinder: Düring, Klaus, 82494 Krün (DE); Dlaikan-Campos, Nasib, 52146 Würselen (DE)
(74) Vertreter: Schüssler, Andrea
(86) Internationale Anmeldenummer: PCT/EP2015/000557
(87) Internationale Veröffentlichungsnummer: WO 2015/135658

(56) Entgegenhaltungen:
- EP-A2- 0 857 471
- WO-A2-2007/065408
- WO-A2-2014/026870
- DE-A1- 19 750 971
- FR-A1- 2 858 208
- US-A1- 2003 040 772
- US-A1- 2005 137 693
- US-A1- 2013 289 690
- US-B2- 6 814 750
- US-B2- 7 655 039

## Beschreibung

Die vorliegende Erfindung betrifft einen komprimierbaren und selbstexpandierenden Stent zur Schienung und/oder Offenhaltung eines Hohlraums, eines Organwegs und/oder eines Gefäßes im menschlichen oder tierischen Körper.

Krankheitsbilder, die auf dem zumindest partiellen Verschluss von Hohlräumen, Organwegen und/oder Gefäßen beruhen, nehmen zivilisationsbedingt stark zu. Ein wichtiger Bereich solcher Krankheitsbilder bezieht sich auf die Atemwege. So ist die obstruktive Schlafapnoe eine lebensbedrohliche Erkrankung, die aus dem Verschluss der hinteren Atemwege durch Muskelerschlaffung während des Schlafes resultiert. Durch wiederholten, teilweise sehr häufigen kurzzeitigen Atemstillstand wird der Patient während des Schlafs nicht ausreichend mit Sauerstoff versorgt, im Extremfall bis hin zur Todesfolge. Zivilisationsbedingte Faktoren wie z.B. starke Fettleibigkeit oder übermäßiger Alkoholgenuss verstärken das Erkrankungsrisiko deutlich. Das Schnarchen ist eine leichtere Effektausprägung als die Schlafapnoe, resultierend aus den gleichen organischen Ursachen. Während das Schnarchen dem Menschen offensichtlich ist, werden die schwerwiegenden gesundheitsgefährdenden Folgen vor allem des starken Schnarchens und der Schlafapnoe oft nicht registriert, da der Patient sich der Folgen der organischen Mangelerscheinungen nicht bewußt wird.

Die Atemstillstände resultieren aus Verschlüssen des Atemweges im Rachenraum, zumeinst im Bereich des Velopharynx, teilweise auch im Bereich des Hypopharynx. Das Ansaugen des Weichgewebes durch entstehenden Unterdruck bei geringem Durchmesser des Atemweges erfolgt zum allergrößten Teil im Velopharynx, weil dort das meiste Weichgewebe vorhanden ist. Demzufolge ist die Schienung des Velopharynx ein entscheidender Schritt für die Schlafapnoe-Therapie.

Aus der WO 2007/065408 A2 geht ein Apnoe-Stent hervor, der zur Schienung und/oder Offenhaltung des Atemwegs im Rachenraum dient. Dieser Apnoe-Stent besteht aus einem komprimierbaren und selbst-expandierenden Stent, der zumindest einen aufgeweiteten Bereich aufweist. Dieser Stent ist dreiphasig ausgebildet. Eine distale Phase des Stents bildet einen funktionellen Teil des Apnoe-Stents aus. Hierbei drückt dieser Bereich des Stents nicht aktiv ein Lumen auf, sondern beim Ansaugen des Weichgewebes durch Unterdruck während der Apnoen und Hypopnoen wird durch den Stent der Verschluß des Atemwegs verhindert, indem sich das angesaugte Weichgewebe den Stent im expandierten Zustand anlegt. Diese distale Phase ist rohrförmig ausgebildet und derart aufweitbar, dass der Atemweg offengehalten wird. Eine proximale Phase des Stents ist zur Fixierung des Stents im Nasenbereich vorgesehen. Die proximale Phase ist trichterförmig ausgebildet, wobei sie sich von einem proximalen zu einem distalen Ende hin aufweitet. Die distale und die proximale Phase sind als Geflecht ausgebildet, bei dem sich Drähte bzw. Fasern bzw. Fäden kreuzen. Jeder einzelne in Richtung zum distalen Ende des Stents verlaufende Draht wird am distalen Ende der distalen Phase zurück zum proximalen Ende des Stents geführt. Die hierdurch erzeugten Biegungen werden als runde Enden bezeichnet. Eine Übergangsphase des Stents ist zum Verbinden der proximalen Phase mit der distalen Phase vorgesehen. Die Übergangsphase ist durch Verzwirbelung der Drähte des Stents zu verzwirbelten Strängen ausgebildet. Hierdurch weist der Übergangsbereich in Radialrichtung eine hohe Flexibilität auf.

In der WO 2012/107229 A2 ist ein Stent zum Schienen eines Nasenganges offenbart. Der Stent besteht aus einem geflochtenen, rohrförmigen Stützkörper, der im unbelasteten Zustand einen Durchmesser von mindestens 4 mm und eine Länge im Bereich von 25 mm bis 100 mm bzw. 25 mm bis 120 mm besitzt. Der Stent kann einen aufgeweiteten Bereich am proximalen Ende aufweisen, um den Nasenflügel zu stützen. Er kann auch alternativ oder in Kombination mit einem Fixierabschnitt versehen sein, der am proximalen Ende des Stents angeordnet und zum Fixieren des Stents in einem Nasengang eines Benutzers ausgebildet ist. Beim Fixieren erstreckt sich der Fixierabschnitt aus dem Nasenloch des Benutzers heraus und ist außerhalb der Nase fixierbar.

In der US 2004/0134491 A1 geht eine Vorrichtung zur Behandlung von Schlafapnoe hervor. Diese Vorrichtung ist von der Größe her zum Platzieren in einer vorbestimmten Position in dem oropharyngealen Bereich angepasst. Die Vorrichtung umfasst eine Schlaufe, die einen Innenraum begrenzt und einen ersten und einen zweiten Endbereich aufweist. Die Schlaufe ist durch zwei langgestreckte Elemente definiert, die voneinander beabstandet angeordnet sind und sich zwischen dem ersten und den zweiten Endbereich erstrecken, wobei die Vorrichtung sich in einer C-förmigen auslandenden Bauform entfaltet, in welcher die langgestreckten Elemente gegen die Seitenwände des oropharyngealen Bereichs drücken und auf diese Weise eine Öffnungskraft auf diesen Bereich ausüben.

In der US 2001/0044647 A1 ist ein modulares endoluminales Stent-Transplantat offenbart. Ein derartiges Stent-Implantat umfasst mindestens zwei Stent-Implantate unterschiedlicher Größe, wobei ein Stent-Implantat innerhalb des anderen entfaltet wird. Das erste Stent-Implantat weist ein chronisch aufgeweitetes Ende auf, dass zu einem Erstdurchmesser expandierbar ist, und einen mittleren Abschnitt der zu einem zweiten Durchmesser expandierbar ist, welcher kleiner als der Erstdurchmesser ist. Das zweite Stent-Implantat weist ein Ende auf, das zu einem Durchmesser expandierbar ist, der in einen mittleren Abschnitt des ersten Stent-Implantats eingreift.

Aus der US 2003/0153973 A1 geht ein geflochtener modularer Stent mit sanduhrförmigen Schnittstellen hervor. Der Stent umfasst eine erste Komponente und eine zweite Komponente, wobei jede Komponente eine stundenglasförmige Grenzfläche aufweist. Hierbei ist vorgesehen, dass ein Abschnitt mit verringertem Durchmesser eine größere radiale Festigkeit aufweist als Abschnitte mit nominellem Durchmesser, wobei der Abschnitt mit verringertem Durchmesser der stundenglasförmigen Grenzfläche ein größeren Flechtwinkel als die Abschnitte mit nominellem Durchmesser aufweist.

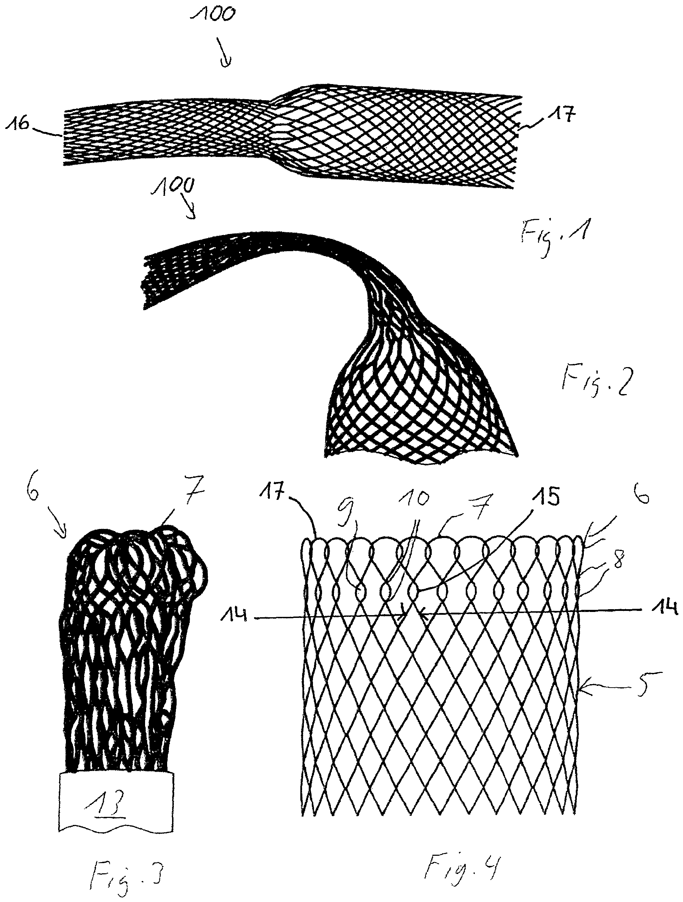

In der DE 2141252 A1 ist ein Naseneinsatz zum Abstützen der Nasenhöhle offenbart, der eine in etwa birnenförmige Form aufweist und aus einem elastisch verformbaren Korb aus Kunststoff ausgebildet ist. Die ungefähre Länge dieses Naseneinsatzes entspricht in etwa der Tiefe der Nasenhöhle des Benutzers und ist anhand der Figur 2 abzuschätzen. Diese zeigt einen Korb der über eine Nasenöffnung in die Nasenhöhle eingesetzt wird.

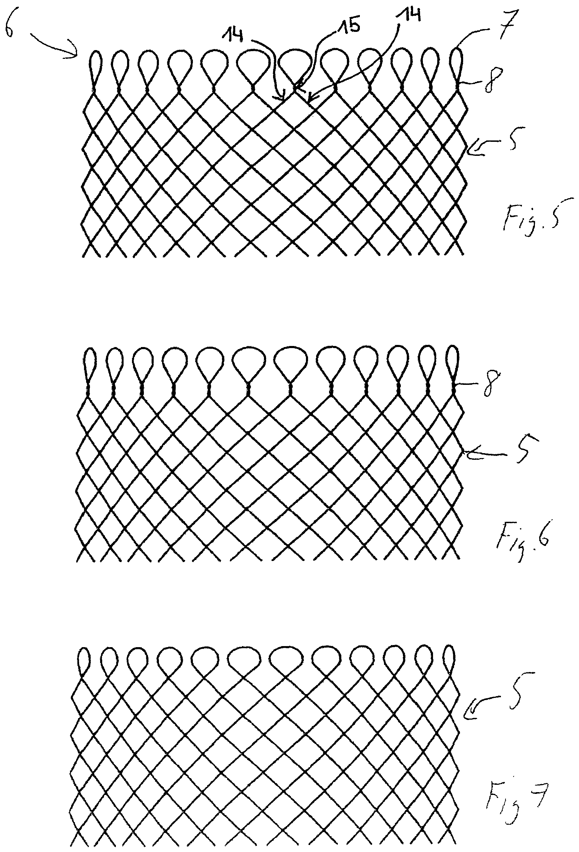

In der WO 03/992765 A2 ist ein selbst expandierbarer Stent zum Einbringen in einen Nasengang zur Behandlung von Apnoe offenbart (Figur 5). Dieser Stent ist derart ausgebildet, dass er sich bis in den Nasenrachenraum des Benutzers erstreckt. Der Stent wird zwischen der Uvula (Gaumenzäpfchen) und der hinteren Pharynxwand im Rachenraum angeordnet. Um den Rachenraum offen zu halten, muss dieser Stent zwangsläufig einen relativ großen Durchmesser aufweisen. Ein distales Ende dieses Stents kann einen sich verjüngenden, in etwa kegelförmig ausgebildeten Abschnitt aufweisen. Weiterhin kann der Stent in etwa zylinderförmig ausgebildet sein. Zudem kann der Stent am proximalen Ende vergrößert sein, um zu Verhindern, dass der proximale Abschnitt in die Nasenhöhle hineinrutscht.

Aus der DE 20 2009 010 388 U1 bzw. der PCT/EP 2010/004687 geht eine Fixiervorrichtung zur Fixierung eines solchen Apnoe-Stents im Atemweg hervor, wobei die Fixiereinrichtung zwei Klemmelemente aufweist, zwischen welchen das proximale Ende des Apnoe-Stents fixierbar ist. Weiterhin ist hierin ein Einführschlauch zum Einführen des Apnoe-Stents beschrieben, der am distalen Ende gebogen ist, um das Einführen in einen Atemweg zu erleichtern. Der Apnoe-Stent weist an seinem proximalen Ende ein Verbindungs- oder Kopplungselement auf, so dass eine Einführstange an das proximale Ende des Apnoe-Stents fixiert werden kann. Mit Hilfe der Einführstange kann der Stent in den Einführschlauch 6 eingeführt und komprimiert werden, indem die Einführstange durch den Einführschlauch geschoben wird, so dass der Apnoe-Stent von der Einführstange in den Einführschlauch gezogen wird.

Aus der US 2009/0010991 A1 geht ein expandierbarer Nasen-Stent hervor, der dauerhaft in die Nasenöffnung bzw. den Nasengang eingesetzt wird. Dieser Stent ist aus einem Kunststoff oder Stahlgitter ausgebildet und mit einem Filterelement versehen, das im proximalen Ende des Stents angeordnet ist. Der Stent kann mit einem Wirkstoff beschichtet sein.

Aus der US 2010/0211181 A1 und der US 5,336,163 gehen weitere Nasen-Stents hervor, die als Filter ausgebildet sind, um die eingeatmete Luft zu filtern.

Die US 2010/0106255 A1 offenbart einen selbst-expandierenden Stent, der mit einem Ende in den Stirnhöhlen verankert werden kann.

In der US 2003/0040772 A1 ist ein Stent beschrieben, der aus mehreren Formgedächtnisdrähten ausgebildet ist, die miteinander verwebt sind. Gemäß diesem Stent ist vorgesehen, dass die Drähte gebogen werden, um gebogene Abschnitte zu erzeugen, wobei die gebogenen Abschnitte derart angeordnet sind, dass sie ein Ende des Körpers definieren. Weiterhin sind Drähte derart gewebt, dass sie einander überkreuzen, um mehrere Winkel auszubilden, wobei wenigstens einer der Winkel stumpf ist. Ein Wert wenigstens eines stumpfen Winkels kann vergrößert werden indem der Körper axial zusammengedrückt wird. Ein derartiger Stent kann an seinen Enden Ösen aufweisen, wobei die Abschnitte des Drahtes, mit denen die Öse geschlossen ist, einander nicht umschlingen und daher nicht verzwirbelt sind.

Aus der DE 10 2009 056 449 A1 geht eine medizinische Vorrichtung hervor, die ein rohrförmiges Gittergeflecht aus Drahtelementen umfasst. An einem distalen Ende des Gittergeflechts sind Endmaschen durch die Drahtelemente ausgebildet, wobei ein erstes Drahtelement einer ersten Endmasche eine Schlaufe mit einer Schlaufenspitze bildet, die mit weiteren Schlaufenspitzen auf einer gemeinsamen, das Gittergeflecht axial begrenzenden Umfangslinie angeordnet ist. Hierbei ist vorgesehen, dass wenigstens ein zweites Drahtelement einer zweiten Endmasche eine Öse durch doppelte Umschlingung des Drahtes um 720° ausbildet, die in axialer Richtung über die Umfangslinie der Schlaufenspitze vorsteht. Im Bereich dieser Öse ist ein Röntgen-Marker-Element angeordnet.

In der US 2005/0283962 A1 ist ein Verfahren zum Flechten eines medizinischen Implantats beschrieben. Hierbei ist vorgesehen, das medizinische Implantat aus einem einzigen Draht auszubilden, wobei an einem oder beiden Endabschnitten des medizinischen Implantates Abschnitte des Drahtes zu einer Öse gebogen sind, wobei die Abschnitte des Drahtes, mit denen die Ösen geschlossen sind, einander nicht umschlingen und daher nicht verzwirbelt sind.

Aus der US 2008/0221670 A1 geht ein radiopaquer Stent hervor. Der Stent ist aus einem Drahtgeflecht ausgebildet, wobei an einem distalen Ende des Stents Ösen ausgebildet sind, wobei die Abschnitte des Drahtes, mit dem die Ösen geschlossen sind, einander nicht umschlingen und daher nicht verzwirbelt sind. Weiterhin ist vorgesehen, dass sich in Längsrichtung erstreckende Abschnitte der Ösen mittels Verschweißen miteinander verbunden sind.

In der EP 1 722 695 B1 ist eine Vorrichtung zur Rekanalisierung eines zumindest teilweise verschlossenen Hohlraumes beschrieben. Die Vorrichtung umfasst zumindest einen komprimierbaren und selbstexpandierenden Stent, der mittels eines Katheters in den Bereich des verschlossenen Hohlraumes einbringbar ist.

In der DE 197 50 971 A1 ist ein Stent zur Implantation in den menschlichen Körper, insbesondere in Blutgefäßen offenbart. Dieser Stent weist einen hohlzylindrischen Körper auf, der aus fadenförmigen, umfänglich zueinander versetzt angeordneten Elementen aufgebaut ist, die ein Geflecht ausbilden. An einem ersten Ende des Stents sind die fadenförmigen Elemente miteinander verdrillt und an einem zweiten Ende sind aus den fadenförmigen Elementen Schlaufen ausgebildet.

Aus der US 6,814,750 B1 geht ein Stent hervor, wobei ein den Stent ausbildender Draht über die Gesamtlänge des Stents in Längsrichtung liegende Verzwirbelungen aufweist. Der den Stent ausbildende Draht verläuft im Wesentlichen quer zur Längsrichtung des Stents. An einem ersten Ende des Stents sind Schlaufen ausgebildet, und an einem zweiten Ende des Stents sind durch den Draht Verzwirbelungen ausgebildet.

In der FR 2 858 208 A1 ist ebenfalls ein Stent offenbart, der aus einem Draht ausgebildet ist, wobei der Stent in Längsrichtung des Stents verlaufende Verzwirbelungen derart aufweist, dass ein Geflecht ausgebildet ist, wobei an einem ersten Ende des Stents Schlaufen angeordnet sind, und an einem zweiten Ende des Stents der Verdrillungen vorgesehen sind.

Aus der EP 0 857 471 A2 geht ein Stent zum Expandieren eines Hohlorgans hervor.

Dieser Stent kann zwei Endabschnitte umfassen, die einen größeren Durchmesser aufweisen als ein zwischen diesen Endabschnitten angeordneter Mittelabschnitt, der einen geringeren Durchmesser aufweist als die Endabschnitte. Weiterhin weist dieser Stent miteinander verzwirbelte Drähte auf, die ein Geflecht ausbilden, wobei die Verzwirbelungen quer zur Längsrichtung des Stents angeordnet sind.

Aus der US 7,655,039 B2 geht ein Stent hervor, an dessen einen Ende Schlaufen ausgebildet sind. Dieser Stent kann einen in etwa zylindrischen, proximalen Abschnitt aufweisen, und einen aufgeweiteten, ebenfalls zylindrisch ausgebildeten distalen Abschnitt. Der den Stent ausbildende Draht weist Verzwirbelungen auf, die in etwa quer zur Längsrichtung des Stents angeordnet sind.

Aus der WO 2014/026870 A2 geht eine implantierbare Einrichtung zum Ersatz einer Organklappe hervor. Dieser Stent weist in Längsrichtung verlaufende Verzwirbelungen auf, die ein in etwa zylindrisches Lumen mit konstantem Durchmesser begrenzen. In diesem Lumen ist eine Membran auangeordnet, um beispielsweise einen Herzklappenersatz nach Art eines Ventils auszubilden. Auf diese Weise soll eine Fluiddichtigkeit der implantierbaren Einrichtung an einem Ende der Einrichtung erreicht werden. Weiterhin weist dieser Stent einen kreisringförmigen Verankerungsabschnitt, der durch einen spiralförmigen Verlauf eines die implantierbare Einrichtung ausbildenden Drahts bereitgestellt wird.

Aufgabe der vorliegenden Erfindung ist es, einen komprimierbaren und selbstexpandierenden Stent zum Schienen und/oder zur Offenhaltung eines Hohlraums, eines Organwegs und/oder eines Gefäßes im menschlichen oder tierischen Körper bereitzustellen, der einfach und zuverlässig expandiert.

Aufgabe der vorliegenden Erfindung ist es zudem, einen Stent zum Schienen des Rachenraums oder eines Nasenganges bereitzustellen, der beim Benutzen, insbesondere beim Einführen, kein unangenehmes Gefühl im Rachenraum bzw. auf der Schleimhaut oder im Nasengang hervorruft.

Eine weitere Aufgabe der vorliegenden Erfindung ist es, zu verhindern, dass ein Abschnitt des Stents im expandierten, in den Rachenraum eingeführten Zustand abknickt.

Diese Aufgaben werden durch einen Stent mit den Merkmalen des Anspruchs 1 gelöst. Vorteilhafte Ausgestaltungen sind in den Unteransprüchen angegeben.

Erfindungsgemäß ist ein komprimierbarer, selbst-expandierender Stent zum Schienen und/oder zur Offenhaltung eines Hohlraums, eines Organwegs und/oder eines Gefäßes im menschlichen oder tierischen Körper vorgesehen. Der Stent ist schlauchförmig ausgebildet und weist ein Geflecht aus zumindest einem Draht auf. Abschnitte des bzw. der Drähte, die sich zwischen einem proximalen und einem distalen Ende des Stents erstrecken werden als Stränge bezeichnet. Am distalen Ende des Stents sind jeweils zwei Stränge mit einem Verbindungsabschnitt verbunden. Ein jeder Verbindungsabschnitt ist zu einer Öse gebogen, wobei jede Öse durch Umschlingung der jeweils zwei Stränge eine Verzwirbelung ausbildet, mit welcher die Öse geschlossen ist.

Ein komprimierbarer und selbstexpandierender Stent zum Schienen und/oder zur Offenhaltung eines Hohlraums, eines Organwegs und/oder eines Gefäßes im menschlichen oder tierischen Körper ist insbesondere als Stent zum Schienen des Rachenraumes oder auch zum Schienen des Nasenganges ausgebildet.

Bei einem Stent, der gemäß der WO 2007/065408 ausgebildet ist, sind am distalen Ende des Stents Biegungen vorgesehen. Diese Biegungen werden als runde Enden bezeichnet. Nachteilig bei einer derartigen Ausbildung des distalen Endes des Stents ist, dass sich das distale Ende des Stents beim Herausführen aus einem Einführschlauch, d.h. unmittelbar (ca. 0,5 bis 1,5 cm) nachdem das distale Ende des Stents den Einführschlauch verlassen hat, stark konisch bzw. trichterförmig aufweitet. Dies führt dazu, dass das distale Ende des Stents in einem schmalen Abschnitt mit einem relativ starken Druck gegen die Schleimhaut in dem Bereich, in dem der erste Kontakt zwischen Schleimhaut und Stent stattfindet, bzw. im oberen Bereich des Rachenraumes, drückt. Dieser Druck führt zu einem starken Reflex, den viele Patienten als Würgereiz empfinden. Dies mindert die Toleranz und Akzeptanz gegenüber einem derart ausgebildeten Stent. Hierbei ist es unerheblich, ob die runden Enden sich in etwa in Längsrichtung erstrecken oder ob diese konvex nach innen gebogen sind.

Dadurch, dass beim erfindungsgemäßen komprimierbaren selbstexpandierenden Stent zum Schienen des Rachenraums gemäß der vorliegenden Erfindung an einem distalen Ende des Stents jeweils ein Abschnitt des Drahtes zu einer Öse gebogen ist, wobei der Abschnitt des Drahtes zu einer Verzwirbelung umschlungen ist, mit welcher die Öse geschlossen ist, wird eine zu stark konische Aufweitung eines distalen Endes des Stents beim Expandieren bzw. beim Herausschieben aus einem Einführschlauch vermieden.

Der Draht oder die Vielzahl von Drähten können sich in einer Längsrichtung des Stents von proximal nach distal und umgekehrt erstrecken. Der Abschnitt eines Drahtes, der sich zwischen einem proximalen Ende des Stents und einem distalen Ende des Stents erstreckt, wird als Strang bezeichnet. Jeder Draht weist zumindest zwei Stränge auf. Ein einzelner Draht kann auch mit mehreren Strängen ausgebildet sein, deren Anzahl vorzugsweise geradzahlig ist, da am distalen Ende keiner der Drähte ein freies Ende bildet. Am distalen Ende des Stents sind zwei Stränge über einen Verbindungsabschnitt verbunden. Es kann ins besonders vorgesehen sein, dass der Stent in jedem Verbindungsabschnitt an seinem distalen Ende eine Öse aufweist. Unter dem Ausdruck "in jedem Verbindungsabschnitt" wird im Rahmen der vorliegenden Erfindung verstanden, dass fast alle bzw. die meisten der Verbindungsabschnitte mit einer Öse versehen sind. Wenn einige wenige Verbindungsabschnitte, z.B. maximal 10% oder maximal 20% der Verbindungsabschnitte, keine erfindungsgemäße Öse aufweisen, dann weitet sich der Sten auch noch etwa zylinderförmig auf. Im Gegensatz dazu weist ein gemäß der DE 10 2009 056 449 A1 ausgebildeter Stent nur eine Öse auf, d.h. es ist nicht an jeden Verbindungsabschnitt eine Öse ausgebildet.

Überraschenderweise hat sich gezeigt, dass durch die Ösen mit Verzwirbelung ein distaler Abschnitt des Stents beim Expandieren sich in etwa parallel bzw. zylindrisch und nicht konisch bzw. trichterförmig aufweitet, wie dies bei aus dem Stand der Technik bekannten Stents der Fall ist. Auch ist der Durchmesser des distalen Abschnittes des erfindungsgemäßen Stents während des Zurückziehens eines Einführschlauches, bei gleicher freiliegender Stentlänge, geringer als bei einem aus dem Stand der Technik bekannten sich trichterförmig aufweitenden Stent.

Im Bereich der Verzwirbelung sind die Stränge nicht zusätzlich mit einer Klammer, einer Lötstelle oder sonstigen weiteren Verbindungen verbunden, so dass die Stränge im Bereich der Verzwirbelung beim Komprimieren des Stents etwas auseinander gehen können.

Auf diese Weise wird verhindert, dass das distale Ende des Stents in die Schleimhaut drückt, weil der erste Kontakt des Stentgeflechts mit der umgebenden Schleimhaut erst dann erfolgt, wenn das zylindrisch öffnende Stentgeflecht einen Großteil seines Durchmessers im unbelasteten Zustand eingenommen hat. In diesem Zustand wird die Radialkraft des Geflechts gleichmäßig über eine größere Fläche und nicht über einen schmalen Abschnitt auf die Schleimhaut übertragen, da kein versteifter ringförmiger Bereich am distalen Ende des Stents im Geflecht ausgebildet wird, wie dies bei nach innen gebogenen distalen Enden der Fall sein kann.

Auf diese Weise wird der Tragekomfort und insbesondere das Einführen für den Patienten erheblich verbessert, wodurch die Toleranz und Akzeptanz unter den Patienten steigt, ohne dass die funktionelle Wirksamkeit eingeschränkt wird.

Die Verzwirbelung benachbart zur Öse kann als halber Schlag ausgebildet sein, wobei der halbe Schlag einer Umschlingung des Drahtes von 180°entspricht.

Bei Versuchen wurde festgestellt, dass diese Effekte nicht auftreten, wenn die Verzwirbelung zumindest als ganzer Schlag oder mehr ausgebildet ist, wobei dies einer Umschlingung des Drahtes von 360° entspricht (Figur 6).

Die oben beschriebenen Effekte treten ebenfalls nicht auf, wenn an einem distalen Ende des Stents ein Abschnitt des Drahtes zu einer Öse gebogen ist, wobei der Abschnitt des Drahtes, mit dem die Öse geschlossen ist, nicht verzwirbelt ist (Figur 7). Bei einer derartigen Ausbildung kommt es beim Herausführen des Stents aus einem Schlauch zu einer stark trichterförmigen Aufweitung und zudem drehen sich die Ösen beim Hineinziehen in einen Einführschlauch schlußendlich radial nach außen und es wird ein noch unangenehmeres Gefühl beim Benutzer durch eine Verletzung der Schleimhaut hervorgerufen.

Beispiele für Ösen ohne Verzwirbelung sind außer in Figur 7 auch den Patentdokumenten US 2003/0040772 A1, US 2005/0283962 A1 und US 2008/0221670 A1 zu entnehmen. Demgemäß stellt eine Öse, bei der ein Abschnitt des Drahtes zu einer Öse gebogen ist, wobei in dem Bereich des Drahtes, in dem die Öse geschlossen ist, sich die Drähte lediglich im Kreuzungspunkt berühren, jedoch nicht umschlingen, keine Verzwirbelung im Sinne der vorliegenden Erfindung dar.

Beim Aufweiten des Stents spielen mehrere Effekte eine Rolle, wobei noch nicht abschließend geklärt werden konnte, welche Bedeutung die einzelnen Effekte für die erläuterte Wirkung haben. Sicher ist jedoch, dass eine Verzwirbelung als halber Schlag, wobei der halbe Schlag einer Umschlingung des Drahtes von 180° entspricht, am vorteilhaftesten ist und auch eine Umschlingung von einem ganzen Schlag, was einer Umschlingung des Drahtes von 360° entspricht, ebenfalls noch geeignet ist.

Es hat sich gezeigt, dass beim Aufweiten des Stents die im Bereich der Verzwirbelung aneinanderliegenden Drahtabschnitte aneinander reiben. Diese Reibung wirkt einer Öffnung des Stents im distalen Endbereich entgegen. Hierdurch wird die Aufweitung am distalen Endbereich verzögert, so dass kein Trichter oder nur ein Trichter mit einem sehr geringen Öffnungswinkel entsteht. Bei einer Umschlingung des Drahtes mit einem halben Schlag sind die Drahtabschnitte im Bereich der Verzwirbelung lateral relativ frei beweglich, so dass hier über einen weiten Bewegungsspielraum ein gleichmäßiger Reibungswiderstand vorliegt.

Es wird auch davon ausgegangen, dass durch das Verdrehen bzw. Verzwirbeln beim Ausbilden der Öse eine Torsionsspannung in die an den verwinkelten Bereich angrenzenden Bereiche des Drahtes eingeleitet wird, welche beim Expandieren des Stents der Expansionskraft entgegenwirkt und diese Torsionsspannung im Draht der Expansionskraft entgegenwirkt und auf diese Weise ebenfalls eine zu starke radiale Aufweitung verhindert wird.

Durch das Verzwirbeln des Abschnitts des Drahtes beim Ausbilden der Öse erfolgt eine lokale Schwächung des Materials bzw. Einschnürung des Drahtes. Diese Einschnürung des Drahtes bewirkt eine stärkere Flexibilität. Da bei einem in einem Einführschlauch zusammengedrückten Stent die einzelnen Drahtabschnitte des Geflechts zusammengedrückt und etwa parallel zueinander ausgerichtet werden, wird eine Spannung im distalen Endbereich des Stents jeweils zwischen zwei Drahtabschnitten, die jeweils über eine Öse miteinander verbunden sind, aufgebaut. Aufgrund des flexiblen Abschnittes im Bereich der Einschnürung können die beiden Drahtabschnitte mit weniger Kraft zueinander gebogen werden, wodurch die Spannungen geringer sind. Eine geringe Spannung im distalen Endabschnitt des Stents bewirkt auch eine nicht so starke Aufweitung.

Es wird davon ausgegangen, dass die oben erläuterten Effekte dazu führen, dass bei der erfindungsgemäßen Ausbildung eine nicht so stark trichterförmige Aufweitung erfolgt.

Somit wird erfindungsgemäß ein komprimierbarer, selbst-expandierender Stent zum Schienen und/oder zur Offenhaltung eines Hohlraums, eines Organwegs und/oder eines Gefäßes im menschlichen oder tierischen Körper bereitgestellt, der insbesondere zur Behandlung von obstruktiver Schlafapnoe (OSA) und von Schnarchen verwendet werden kann. Hierbei ist es vorteilhaft, da der Stent vom Patienten selbst jeden Abend vor dem Schlafen eingesetzt und morgens wieder entfernt werden muss, dass dieser Vorgang möglichst schmerzfrei, d.h. ohne größere Reizung des entsprechenden Körpergewebes, ausführbar ist. Dies ist auf Grund der vorstehend beschriebenen Effekte, der zylinderförmigen Aufweitung, mittels des erfindungsgemäßen Stents möglich.

Im nicht expandierten Zustand des Stents kann der Abschnitt des Drahtes, der zu einer Verzwirbelung umschlungen ist bzw. in dem die aneinanderliegenden Drahtabschnitte aneinander reiben, derart aufgeweitet sein, dass im Bereich der Verzwirbelung ein elliptisch aufgeweiteter Abschnitt ausgebildet ist.

Der Stent kann einen distalen Abschnitt, einen proximalen Abschnitt und einen zwischen diesen beiden ausgebildeten Übergangsabschnitt umfassen, wobei der distale Abschnitt eine größere Aufweitung als der proximale Abschnitt aufweist.

Vorzugsweise können die Ösen am distalen Ende um 40° bis 55° und insbesondere von 45° gegenüber der Axialrichtung konisch zulaufend nach innen abgewinkelt sein, so dass die Ösen einen konisch zulaufenden distalen Randbereich des Stents ausbilden.

Weiterhin kann in einem distalen Bereich des distalen Abschnittes, im zu den Ösen benachbarten Bereich, das Geflecht in distaler Richtung verjüngend ausgebildet sein. Der Winkel dieses konisch bzw. verjüngend zulaufenden Bereichs gegenüber der Axialrichtung beträgt 2° bis 20° bzw. 15° bzw. 10° und insbesondere in etwa 5° bis 7,5°.

Der Stent kann auch rohrförmig mit in etwa konstantem Durchmesser ausgebildet sein.

In beiden Fällen ist der Stent elastisch verformbar und schmiegt sich an die Innenfläche des Rachenraums an.

Weiterhin ist erfindungsgemäß ein komprimierbarer, selbst-expandierender Stent zum Schienen und/oder zur Offenhaltung eines Hohlraums, eines Organwegs und/oder eines Gefäßes im menschlichen oder tierischen Körper, insbesondere des Rachenraums vorgesehen. Der Stent ist aus zumindest einem Draht ausgebildet und umfasst einen distalen Abschnitt, einen proximalen Abschnitt und einen zwischen diesen beiden ausgebildeten Übergangsabschnitt, wobei der distale Abschnitt eine größere Aufweitung als der proximale Abschnitt aufweist. Die Abschnitte weisen ein Geflecht auf. Im zum Übergangsabschnitt benachbarten Bereich des proximalen Abschnitts ist ein Entkopplungsbereich ausgebildet, in dem jeweils mindestens zwei Abschnitte des zumindest einen Drahtes bzw. der Drähte miteinander verzwirbelt sind.

Bei einem gemäß der WO 2007/065408 ausgebildeten Stent ist eine Übergangsphase vorgesehen, in der jeweils mindestens zwei Abschnitte des zu zumindest einen Drahtes miteinander verzwirbelt sind. Ein derartiger Stent ist in expandiertem, nicht-eingeführtem Zustand in Figur 1 dargestellt. Nachteilig bei einem derartigen Stent ist, dass das Geflecht des Stents im Bereich benachbart zur Verzwirbelung im eingeführten Zustand beim Übergang vom Nasengang in den Rachenraum (Nasopharynx) durch die Biegung sehr häufig abknickt bzw. zusammenklappt (Figur 2). Dies hat zur Folge, dass in diesem gebogenen Bereich ein relativ breiter, flacher und daher steifer Bereich ausgebildet wird, der gegen die Schleimhaut an der oberen rückwärtigen und oberen horizontalen Wand des Nasenrachenraums drückt. Da dieser Bereich relativ empfindlich ist, mindert diese Eigenschaft die Akzeptanz eines derartigen Stents bei Patienten.

Dadurch, dass im zum Übergangsabschnitt benachbarten Bereich des proximalen Abschnittes ein Entkopplungsbereich ausgebildet ist, in dem jeweils mindestens zwei Abschnitte des zumindest einen Drahtes bzw. der Drähte miteinander verzwirbelt sind, wird verhindert, dass der Übergangsabschnitt den in den Nasengang eingeführten abgewinkelten Zustand kollabiert bzw. zusammengedrückt wird. Durch den Entkopplungsbereich kommt es sogar zu einer lokalen Aufweitung im Übergangsabschnitt derart, dass der Durchmesser des Übergangsabschnittes im expandierten, abgewinkelten Zustand annähernd dem Durchmesser des proximalen Abschnitts entspricht und somit aufgeweitet wird und nicht seine trichterförmige Form behält.

Auf diese Weise wird ab dem Entkopplungsbereich in distaler Richtung ein Bereich mit in etwa gleichbleibendem Durchmesser des Geflechts ausgebildet, wobei dieser eine in etwa gleichbleibende Radialkraft aufweist. Zudem wird im äußeren, an die Schleimhaut anliegenden Bereich eine sehr gleichmäßige Krümmung erzielt. Das Geflecht drückt in diesem Bereich nicht mit einem engen Bereich stärker gegen die Schleimhaut als angrenzende Bereiche. Auf diese Weise wird das Öffnen des Stents so gut wie gar nicht mehr wahrgenommen.

Die Verzwirbelung zweier Abschnitte kann zumindest als ganzer Schlag ausgebildet sein, wobei der ganze Schlag einer Umschlingung bzw. Verzwirbelung der zwei Abschnitte des Drahtes von 360° entspricht. Vorzugsweise sind zumindest 1 ½ bzw. zumindest 2 bzw. zumindest 2 ½ bzw. zumindest 3 Schläge vorgesehen.

Das Geflecht kann eine Vielzahl von Öffnungen aufweisen, wobei die Öffnungen im Übergangsabschnitt größer sind als die übrigen Öffnungen des proximalen Abschnitts. Dadurch wird die Angleichung der Radialkraft im Übergangsabschnitt im gebogenen Zustand an diejenige des distalen Abschnitts erreicht und das Geflecht nimmt im Übergangsabschnitt im expandierten gebogenen Zustand eine aufgeweitete Form ein.

Der aufgeweitete Bereich des Geflechts im distalen Abschnitt sollte für einen gleichmäßigen Druck gegen die Schleimhaut annähernd gleich große Rauten aufweisen. Im Übergangsabschnitt sollten die Rauten deutlich größer sein, da ansonsten dort die Radialkraft deutlich ansteigen und für den Patienten unangenehm hoch würde.

Der verzwirbelte Entkopplungsbereich dient zur funktionalen Trennung von distal aufgeweitetem Abschnitt mit hoher Radialkraft und dem proximal längeren Abschnitt mit geringerer Radialkraft. Durch die Verzwirbelung wird verhindert, dass sich unkontrolliert Kräfte aus dem distalen Abschnitt des Geflechts in den proximalen Abschnitt des Geflechts übertragen und umgekehrt und daraus unerwünschte und die Funktionalität des Stents beeinträchtigende Verschiebungen im Geflecht resultieren.

Eine gleichmäßige Krümmung im Bereich der Schleimhaut an der oberen rückwärtigen und oberen horizontalen Wand des Nasenrachenraums wird somit dadurch erzielt, dass die Verzwirbelung bzw. der Entkopplungsbereich im zum Übergangsabschnitt benachbarten Bereich des proximalen Abschnittes (mit dem geringeren Durchmesser) ausgebildet ist. Der Abstand der Verzwirbelung im proximalen Abschnitt vom Übergangsabschnitt beträgt in proximaler Richtung zumindest ca. 0,2 bis 2,5 cm und vorzugsweise zumindest ca. 0,5 cm bis 1,5 cm.

Weiterhin ist erfindungsgemäß eine Vorrichtung zur Schienung und/oder Offenhaltung des Rachenraums mit zumindest einem in einem Einführschlauch komprimierbaren und expandierenden Stent vorgesehen.

Ein Abschnitt des Einführschlauchs kann kreissegmentförmig gebogen sein, um das Einführen in den Rachenraum zu erleichtern. Der kreissegmentförmig gebogene Abschnitt weist eine Biegung von etwa 80° bis 120° auf.

Die nachfolgenden Ausführungen gelten für die vorstehend beschriebenen erfindungsgemäßen Einrichtungen gleichermaßen, sofern geeignet.

Der Stent kann aus Drähten, Fasern und/oder Fäden, die sich kreuzen, geflochten sein. Im Folgenden wird lediglich der Begriff Draht verwendet, wobei hiermit auch Fasern oder Fäden gemeint sind, sofern der Draht nicht näher definiert ist.

Der Draht, aus dem der Stent geflochten ist, ist insbesondere ein Metalldraht, vorzugsweise ein Nitinoldraht oder ein Stahldraht.

Der Stent ist elastisch verformbar und schmiegt sich an die Innenfläche des Nasenganges an. Der vom Stent auf den Nasengang erzeugte Druck verteilt sich gleichmäßig, wodurch der Stent in dem empfindlichen Rachenraum keine Schmerzen, Reflexe oder unangenehme Gefühle verursacht. Da der Stent geflochten ist, schmiegt er sich an unterschiedliche Formen des Rachenraums an und es ist nicht notwendig, den Stent an den Rachenraum eines bestimmten Benutzers individuell anzupassen. Mit einigen wenigen Standardgrößen des Stents, die sich vor allem in der Länge und im Durchmesser des schlauchförmigen Stents bzw. des distalen, des proximalen und des zwischen diesen beiden ausgebildeten Übergangsabschnitts unterscheiden, bevorzugt mit einer einzigen Standardgröße, kann für fast jede Person ein geeigneter Stent vorgesehen werden.

Die Erfindung wird nachfolgend beispielhaft anhand der Zeichnungen näher erläutert. Die Zeichnungen zeigen in:
- Fig. 1: eine seitliche schematische Ansicht eines komprimierbaren, selbstexpandierenden Stents zum Schienen eines Nasenganges gemäß dem Stand der Technik im nicht-expandierten, nicht-abgeknickten Zustand,
- Fig. 2: den Stent aus Figur 1 im, in den Körper eingeführten, abgeknickten Zustand,
- Fig. 3: eine seitliche schematische Ansicht eines distalen Endbereichs eines nichtexpandierten, bis auf diesen distalen Endbereich vollständig in einem Einführschlauch angeordneten Stents gemäß der vorliegenden Erfindung,
- Fig. 4: eine seitliche schematische Ansicht eines distalen Abschnitts eines erfindungsgemäßen Stents im nicht vollständig expandierten Zustand,
- Fig. 5: den distalen Abschnitt des erfindungsgemäßen Stents aus Figur 4 im vollständig expandierten Zustand,
- Fig. 6: eine seitliche schematische Ansicht eines distalen Abschnitts eines Stents, wobei Ösen durch mehrfache Verzwirbelung ausgebildet sind,
- Fig. 7: eine seitliche schematische Ansicht eines distalen Abschnitts eines Stents, wobei Ösen ohne Verzwirbelung ausgebildet sind,
- Fig. 8: eine seitliche schematische Ansicht eines erfindungsgemäßen Stents, mit proximalen Abschnitt, Entkopplungsbereich, Übergangsabschnitt und distalem Abschnitt,
- Fig. 9: den Stent aus Figur 8 im expandierten, gebogenen bzw. in den Körper eingeführten Zustand, und
- Fig. 10: eine seitliche schematische Ansicht eines erfindungsgemäßen Stents mit allen Abschnitten.

Im Folgenden wird ein komprimierbarer, selbst-expandierender Stent 1 zum Schienen des Rachenraums und insbesondere ein erfindungsgemäßes distales Ende für einen komprimierbaren, selbst-expandierenden Stent 1 zum Schienen des Rachenraums beschrieben.

Der Stent 1 kann, sofern nichts anderes beschrieben ist, ähnlich den in der WO 2007/065408 (Fig. 1) ausgebildeten Stents ausgebildet sein, auf die hiermit vollinhaltlich Bezug genommen wird.

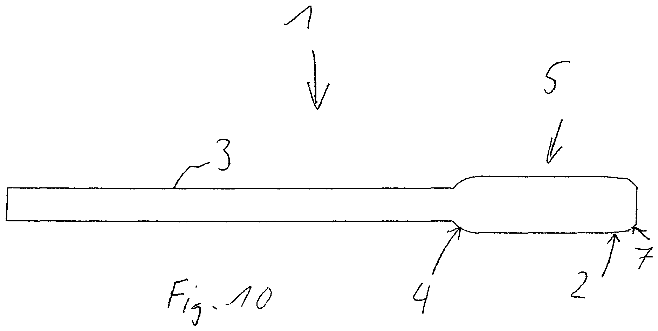

Der Stent 1 zum Schienen eines Rachenraumes ist dreiphasig ausgebildet und weist einen proximalen Abschnitt 3, einen Übergangsabschnitt 4 und einen distalen Abschnitt 5 auf (Fig. 10). Der proximale Abschnitt 3 ist in etwa rohrförmig mit konstantem Durchmesser ausgebildet, wobei an dessen proximalem Endbereich der Draht zusammengefasst ist. Dieser Bereich bildet eine Art Fixierabschnitt aus. Der Übergangsabschnitt 4 ist in distaler Richtung konisch aufweitend ausgebildet. Der distale Abschnitt 5 ist rohrförmig mit in etwa konstantem Durchmesser ausgebildet.

Alternativ ist denkbar, dass der Stent insbesondere zur Schienung eines Nasenganges einen Stützkörper umfasst, der in etwa rohrförmig mit konstantem Durchmesser ausgebildet ist, wobei ein Fixierabschnitt sich von seinem distalen Ende in Richtung zu seinem proximalen Ende konisch verjüngt (nicht dargestellt). Sowohl der Stützkörper als auch der Fixierabschnitt sind integral aus einem Drahtgeflecht ausgebildet

Der Stent 1 kann aus einem einzigen Draht oder aus einer Vielzahl von Drähten geflochten sein. Die Enden des Drahtes bzw. der Drähte sind am proximalen Ende des Stents bzw. am proximalen Ende des Fixierabschnitts bzw. des proximalen Abschnitts mit einem stiftförmigen bzw. zylinderförmigen Körper (nicht dargestellt) zusammengefasst. Der Stent ist aus einem elastischen Draht mit einem Durchmesser von 0,001 mm bis 2 mm, insbesondere mit einem Durchmesser von 0,05 mm bis 0,5 mm und vorzugsweise mit einem Durchmesser von 0,07 mm bis 0,2 mm, am meisten bevorzugt 0,10 bis 0,15 mm geflochten.

Der Draht oder die Vielzahl von Drähten erstrecken in einer Längsrichtung des Stents 1 von proximal nach distal und umgekehrt. Der Abschnitt eines Drahtes, der sich zwischen einem proximalen Ende 16 des Stents und einem distalen Ende 17 des Stents 1 erstreckt, wird als Strang 14 bezeichnet. Jeder Draht weist zumindest zwei Stränge 14 auf. Ein einzelner Draht kann auch mit mehreren Strängen 14 ausgebildet sein, deren Anzahl vorzugsweise geradzahlig ist, da am distalen Ende keiner der Drähte ein freies Ende bildet. Am distalen Ende des Stents 1 sind zwei Stränge 14 über einen Verbindungsabschnitt 15 verbunden. Ein jeder Verbindungsabschnitt 15 ist zu einer Öse 7 gebogen, wobei jede Öse 7 durch Umschlingung der jeweils zwei Stränge 14 eine Verzwirbelung 8 ausbildet, mit welcher die Öse 7 geschlossen ist

Erfindungsgemäß ist an dem distalen Ende 6 des Stents 1 jeweils ein Abschnitt des Drahtes zu einer Öse 7 gebogen (Fig. 3 bis 5). Dieser Abschnitt des Drahtes ist zu einer Verzwirbelung 8 umschlungen, mit welcher die Öse 7 geschlossen ist. Auf diese Weise wird jeder einzelne, in Richtung zum distalen Ende des Stents 1 verlaufende Draht am distalen Ende zurück zum proximalen Ende des Stents geführt. Somit sind radial umlaufend am distalen Ende des Stents mehrere Ösen 7 angeordnet.

D.h. vorzugsweise ist an jedem Abschnitt des Drahtes bzw. an jedem gebogenen Drahtabschnitt, d.h. an jedem Verbindungsabschnitt 15, der am distalen Ende des Stents ausgebildet ist, eine Öse 7 vorgesehen.

Somit weist der Stent 1 an seinem distalen Ende radial umlaufend gleichbeabstandet voneinander angeordnete Ösen 7 auf. Die Ösen 7 sind im nicht-expandierten Zustand in etwa fächerförmig bzw. blütenartig, zylindrisch einander überlappend derart angeordnet, dass keine Abschnitte der Ösen 7 hervorstehen.

Der Radius der Ösen 7 am distalen Ende im expandierten Zustand des Stents 1 liegt im Bereich von ca. 0,5 mm bis 2,0 mm bzw. von 0,7 mm bis 1,0 mm. Auf diese Weise wird ein distales Ende 6 bereitgestellt, an dem keine einzelnen Drahtenden freiliegen, wobei durch die Ösen und die Verzwirbelung 8 insbesondere auch eine Aufweitung des distalen Endes 6 beim Expandieren des Stents durch Zurückziehen eines Einführschlauchs verhindert wird. Durch die verzwirbelten Ösen 7 werden Reizungen der Schleimhaut im Rachenraum durch das distale Ende 6 des Stents insbesondere dadurch vermieden, dass durch die Ösen mit Verzwirbelung ein distaler Abschnitt des Stents beim Expandieren sich in etwa parallel bzw. zylindrisch und nicht konisch aufweitet.

Die Verzwirbelung 8 ist vorzugsweise als halber Schlag ausgebildet, wobei der halbe Schlag einer Umschlingung des Drahtes von 180° entspricht.

Im nicht-expandierten Zustand des Stents ist derjenige Abschnitt des Drahtes, der zu einer Verzwirbelung 8 umschlungen ist, derart aufgeweitet, dass im Bereich der Verzwirbelung ein elliptisch aufgeweiteter Abschnitt 9 ausgebildet ist.

Im elliptisch aufgeweiteten Abschnitt bildet der Draht zwei Reibungsabschnitte 10, in denen jeweils zwei Abschnitte des Drahtes kontaktieren.

Die Ösen 7 oder die Ösen 7 und die Verzwirbelung 8 können sich in etwa in Längsrichtung erstrecken oder konisch zulaufend ausgebildet sein. In dem Falle, dass die Ösen 7 oder die Ösen 7 und die Verzwirbelung 8 konisch zulaufend ausgebildet sind, beträgt der Winkel zwischen Öse und Längsrichtung in etwa 40° bis 60° und vorzugsweise zwischen 45° und 50°.

Die Länge der Ösen 7 in axialer Richtung beträgt im in Längsrichtung erstreckenden Zustand ca. 2,5 mm bis 3,5 mm und insbesondere 3 mm und im abgewinkelten Zustand ca. 1,5 mm bis 2,5 mm und insbesondere 2 mm.

In einem distalen Bereich 2 des distalen Abschnittes, im zu den Ösen benachbarten Bereich, ist das Geflecht konisch zulaufend ausgebildet sein. Der Winkel dieses konisch zulaufenden Bereichs gegenüber der Axialrichtung beträgt 2° bis 20° bzw. 15° bzw. 10° und insbesondere in etwa 5° bis 7,5°. Die Länge dieses distalen Bereichs 2 beträgt ca. 4-5 mm bis 7-8 mm und insbesondere 6 mm.

Bei einer weiteren im Folgenden beschriebenen Ausführungsform eines erfindungsgemäßen Stents können die vorstehend beschriebenen Merkmale zusätzlich oder alternativ vorgesehen sein.

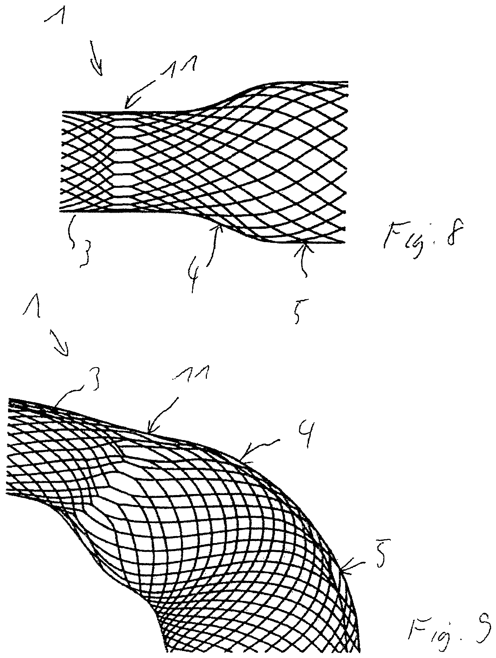

Bei dieser weiteren Ausführungsform des komprimierbaren, selbst-expandierenden erfindungsgemäßen Stents, der den proximalen Abschnitt 3, den Übergangsabschnitt 4 und den distalen Abschnitt 5 aufweist, ist im zum Übergangsabschnitt 4 benachbarten Bereich des proximalen Abschnitts 3 ein Entkopplungsbereich 11 ausgebildet (Fig. 8, 9 und 10).

Der Entkopplungsbereich 11 ist um ca. 0,2 cm bis 2,5 cm, bevorzugt um 0,5 cm bis 1,5 cm in proximaler Richtung bzgl. des Übergangsabschnittes 4 im proximalen Abschnitt 3 in proximaler Richtung zurückversetzt. Vorzugsweise beträgt die Anzahl der Verzwirbelungen im Entkopplungsbereich zumindest 1,5 bzw. zumindest 2 bzw. zumindest 2,5 bzw. zumindest 3.

Im Entkopplungsbereich verlaufen die einzelnen Stränge etwa in Längsrichtung des Stents 1, ohne sich zu kreuzen. Hierdurch weist der Entkopplungsbereich in Radialrichtung eine hohe Flexibilität auf. Dadurch wird sichergestellt, dass über die gesamte Länge des distalen Abschnitts in etwa ein konstanter Durchmesser ausgebildet wird.

Im in den Körper bzw. in den Rachenraum eingebrachten expandierten Zustand ist ein Abschnitt des Stents, der einen proximalen Bereich des proximalen Abschnitts 3, den Übergangsabschnitt 4 und einen distalen Bereich des distalen Abschnitts 5 umfasst, kreissegmentförmig gebogen. Dieser Abschnitt weist dann eine Biegung von etwa 80° bis 120° auf.

Durch den Entkopplungsbereich wird sichergestellt, dass bei einer derartigen Biegung ein distaler Bereich des proximalen Abschnitts 3 bzw. ein proximaler Bereich des Übergangsabschnitts 4 nicht zusammenfaltet bzw. kollabiert, sondern in einem zylinderförmig aufgeweiteten bzw. leicht kugelförmig aufgeweiteten Zustand verbleibt.

Eine gleichmäßige Krümmung des Stents an der oberen rückwärtigen und oberen horizontalen Wand des Nasenrachenraums wird somit dadurch erzielt, dass die Verzwirbelung bzw. der Entkopplungsbereich im zum Übergangsabschnitt benachbarten Bereich des proximalen Abschnittes (mit dem geringeren Durchmesser) ausgebildet ist.

Im Folgenden folgen werden weitere Merkmale des Stents 1 erläutert.

Der distale Abschnitt 5 des Stents bildet einen funktionalen Teil des Apnoe-Stents 1 aus. Der distale Abschnitt 5 ist rohrförmig ausgebildet und derart aufweitbar, dass der Atemweg im Rachenraum offen gehalten wird. Sein Durchmesser beträgt im expandierten Zustand in etwa 12 mm bis 25 mm und vorzugsweise 15 mm bis 18 mm. Der distale Abschnitt 5 weist eine Länge von ca. 30 bis 60 mm bzw. von ca. 40 mm bis 50 mm auf.

Der proximale Abschnitt 3 weist eine Länge von ca. 60 mm bis 150 mm bzw. von ca. 80 mm bis 110 mm auf.

Ein sich an den proximalen Abschnitt anschließender Fixierabschnitt weist eine Länge von etwa 10 bis 40 mm auf. Wobei dieser Abschnitt teilweise oder ganz aus der Nase herausgeführt sein kann. Mit dem Fixierabschnitt kann der Stent derart am Körper des Benutzers fixiert werden, dass der Stent nicht aus dem Nasengang des Benutzers in Richtung zum Rachen verschoben werden kann. Hierzu ist es notwendig, dass der Fixierabschnitt sich durch die Nasenöffnung nach außen erstreckt, um dann dort mit einem entsprechenden Fixierelement fixiert zu werden. Der Fixierabschnitt ist somit zur Fixierung des Stents im Nasenbereich vorgesehen und ist trichterförmig ausgebildet, wobei sie sich von einem proximalen zu einem distalen Ende hin aufweitet.

Der Übergangsabschnitt des Stents ist zum Verbinden des proximalen Abschnitts mit dem distalen Abschnitt vorgesehen. Der Übergangsabschnitt 3 weist eine Länge von ca. 4 mm bis 15 mm bzw. von ca. 7 mm bis 9 mm auf.

Der gesamte Stent weist eine Länge von 10 cm bis 35 cm bzw. von 12 cm bis 20 cm auf.

Der distale, der proximale und der Übergangsabschnitt sind als Geflecht ausgebildet, bei dem sich Drähte bzw. Fasern bzw. Fäden kreuzen. Im Folgenden wird lediglich der Begriff Draht verwendet, wobei hiermit auch Fasern oder Fäden gemeint sind.

Der Draht hat vorzugsweise einen Durchmesser von 0,001 mm bis 2 mm, insbesondere einen Durchmesser von 0,05 mm bis 0,5 mm und vorzugsweise einen Durchmesser von 0,07 mm bis 0,2 mm, am meisten bevorzugt von 0,10 bis 0,15 mm.

Der Stent ist aus einem Formgedächtnismaterial hergestellt. Das Formgedächtnismaterial ist ein biokompatibles Formgedächtnismaterial, insbesondere ein Metall oder eine Metalllegierung, insbesondere Edelstahl oder Nitinol oder einem anderen biokompatiblen Material wie Kunststoff oder monofile und/oder multifile und/oder komposite Glasfasern.

Am proximalen Ende 12 - des Apnoe-Stents 1 ist ein Verbindungs- oder Kopplungselement (nicht dargestellt) angeordnet, das mit einem Verbindungs- oder Kopplungselement einer Einführstange (nicht dargestellt) koppelbar ist. Das Verbindungselement ist an einem Ende der Einführstange angeordnet. Im vorliegenden Ausführungsbeispiel ist das Verbindungselement der Einführstange als Buchse und das Verbindungselement des Stents 1 als formschlüssig in die Buchse passende Kugel ausgebildet.

Zum Einführen des Stents 1 durch einen Nasengang in den Rachenraum wird der Stent 1 mit Hilfe der Einführstange in einen Einführschlauch eingeführt und komprimiert. Hierzu wird die Einführstange durch den Einführschlauch geschoben, so dass der Stent 1 von der Einführstange in den Einführschlauch gezogen wird.

Für die Anwendung dieser Vorrichtung zur Behandlung von Schnarchen und/oder Schlafapnoe wird der Stent 1 in im Einführschlauch komprimierten Zustand in eines der beiden Nasenlöcher eingeführt und in den Velopharynx vorgeschoben und an seinem Anwendungsort platziert. Durch Zurückziehen des Einführschlauches wird der Stent 1 frei gesetzt und expandiert selbstständig in seine vorbestimmte Größe. Dadurch wird das Zusammenfallen des Schlundes und das Verschließen der Atemwege verhindert. Ein freier Luftstrom und eine normale Atmung wird dadurch ermöglicht. Nach der korrekten Anordnung des Stents 1 im Atemweg wird die Verbindung mit der Einführstange gelöst.

Dieser Stent 1 wird mittels einer Fixiervorrichtung (nicht dargestellt) gegen ein zu tiefes Eindringen in und ein Herausrutschen aus dem Atemweg gesichert bzw. fixiert. Mit dieser kann die korrekte Einführungstiefe des Apnoe-Stents festgelegt werden und der Apnoe-Stent 1 gegen unbeabsichtigtes Hineinrutschen in den Atemweg gesichert werden.

Über dem Apnoe-Stent 1 ist während des Einführens der Einführschlauch angeordnet, um den Apnoe-Stent im Atemweg anzuordnen. Der Einführschlauch weist an seinem distalen Ende vorzugsweise einen etwa kreissegmentförmig gebogenen Abschnitt auf, der sich etwa über einen Winkelbereich von ca. 80° bis 120° mit einem Radius von ca. 3 cm bis 7 cm erstreckt. Vorzugsweise ist der Einführschlauch aus einem Zweischichtschlauch mit einem inneren ausreichend harten, geeigneten Kunststoff wie z.B. PEBAX mit einer Shore Härte von 60 Shore D bis 80 Shore D und einem äußeren ausreichend weichen Kunststoff wie z.B. Polyurethan mit einer Shore Härte von 25 Shore D bis 60 Shore D ausgebildet. Die Härte des Kunststoffs hat erheblichen Einfluss auf eine optimale bzw. exakte Anpassung des Einführschlauchs an den Nasengang und die Führbarkeit hinab in den Velopharynx während des Einführvorgangs. Am distalen Ende des Einführschlauchs ist eine Einführspitze einstückig angeformt, die sowohl im Innenals auch im Außendurchmesser dem Einführschlauch entspricht und mit diesem bündig ausgebildet ist. Die Einführspitze kann aus einem flexiblen, wesentlich weicheren Material, wie z.B. Polyurethan mit einer Shore Härte von 25 Shore D bis 45 Shore D ausgebildet sein. Der Einführschlauch 6 kann auch einteilig ausgebildet sein. Ein solcher einteiliger Einführschlauch kann aus einem einheitlichen Material bestehen oder es ist möglich, einen solchen einteiligen Einführschlauch mit einem Extrusionsverfahren mit kontinuierlichem Materialgradienten herzustellen. Bei einem solchen Einführschlauch gibt es einen Übergangsbereich, in dem kontinuierlich der Anteil des einen Materials abnimmt und dementsprechend der Anteil des anderen Materials zunimmt. Die Materialien eines solchen Schlauches sind z.B. PEBAX72 im Bereich des Hauptkörpers und PEBAX35 im Bereich der Einführspitze. Bzgl. der Materialauswahl gelten die obigen Ausführungen zum zweiteiligen Einführschlauch gleichermaßen.

Am distalen Ende der Einführspitze des Einführschlauchs ist eine atraumatische Spitze, bevorzugt als Rundung, vorgesehen. Eine solche Rundung kann außenseitig und/oder innenseitig ausgebildet sein. Auf diese Weise werden Verletzungen der Gefäßwandungen beim Einführen des Apnoe-Stents 1 in den Atemweg vermieden und das Einführen wird erleichtert, da der Einführschlauch ideal den Biegungen des Atemwegs folgen kann und die Einführspitze sehr weich und flexibel ist. Der Außendurchmesser des Einführschlauchs beträgt bis zu 5 mm, bevorzugt etwa 4 mm, um dem Benutzer eine komfortable, schmerzfreie Einführung zu ermöglichen. Der Innendurchmesser des Einführschlauchs beträgt bis zu 4 mm, um ausreichend Raum zur Aufnahme des Apnoe-Stents bereitzustellen. Durch die Krümmung des Einführschlauchs wird ein Anstoßen am Rachenraum verhindert und somit ein verletzungsfreies Einführen sichergestellt.

An Stelle der Rundung kann die Einführspitze auch mit einer Fase, bevorzugt mit einem Winkel von 30° bis 60°, noch mehr bevorzugt mit einem Winkel von 45°, ausgebildet sein.

Eine solche abgerundete Spitze ist in der Herstellung aufwändiger als eine Fase. Die abgerundete Spitze ist jedoch noch sicherer beim Einführen des Stents. Zudem kann bei einer Fase das sich ergebende dünnwandige Ende verhärten, indem die Weichmacher aus dem Kunststoffmaterial durch die relativ große Oberfläche entweichen.

Diese geformten Einführspitzen stellen atraumatische Spitzen dar, wobei je härter das Material der Spitze ist, desto glatter und runder sollte die Form sein.

Alle in dieser Anmeldung gemachten Längenangaben sind Durchschnittswerte. Je nach dem ob es sich um Kinder oder groß gewachsene Personen handelt, können diese Werte dementsprechend variieren.

### Bezugszeichenliste

- 1: Stent
- 2: distaler Bereich
- 3: proximaler Abschnitt
- 4: Übergangsabschnitt
- 5: distaler Abschnitt
- 6: distales Ende
- 7: Öse
- 8: Verzwirbelung
- 9: elliptisch aufgeweiteter Abschnitt
- 10: Reibungsbereich
- 11: Entkopplungsbereich
- 12: proximales Ende
- 13: Einführschlauch
- 14: Strang
- 15: Verbindungsabschnitt
- 16: proximales Ende
- 17: distales Ende
- 100: Stent gemäß dem Std. d. T.

## Patentansprüche

1. Komprimierbarer, selbstexpandierender Stent zum Schienen und/oder zur Offenhaltung eines Hohlraums, eines Organwegs und/oder eines Gefäßes im menschlichen oder tierischen Körper, wobei der Stent aus zumindest einem Draht ausgebildet ist und einen distalen Abschnitt, einen proximalen Abschnitt und einen zwischen diesen beiden ausgebildeten Übergangsabschnitt umfasst, wobei der distale Abschnitt eine größere Aufweitung als der proximale Abschnitt aufweist, und die Abschnitte ein Geflecht aufweisen, und wobei Abschnitte des Drahtes bzw. der Drähte, die sich zwischen einem proximalen und einem distalen Ende des Stents erstrecken, als Stränge bezeichnet werden, wobei im zum Übergangsabschnitt in proximaler Richtung benachbarten Bereich des proximalen Abschnittes ein Entkopplungsbereich ausgebildet ist, in dem zumindest zwei Stränge miteinander verzwirbelt sind,
**dadurch gekennzeichnet,**
**dass** diese Stränge in etwa in Längsrichtung des Stents verlaufen.

2. Komprimierbarer, selbstexpandierender Stent nach Anspruch 1,
**dadurch gekennzeichnet,**
**dass** die Stränge, die im Entkopplungsbereich in etwa in Längsrichtung des Stents verlaufen, den distalen Abschnitt derart mit konstantem Durchmesser ausbilden, dass dieser in etwa rohrförmig ist.

3. Komprimierbarer, selbstexpandierender Stent nach Anspruch 1 oder 2,
**dadurch gekennzeichnet,**
**dass** am distalen Ende des Stents zwei Stränge über einen Verbindungsabschnitt verbunden sind und ein jeder Verbindungsabschnitt zu einer Öse gebogen ist, wobei jede Öse durch Umschlingung der jeweils zwei Stränge eine Verzwirbelung ausbildet, mit welcher die Öse geschlossen ist.

4. Komprimierbarer, selbstexpandierender Stent nach einem der Ansprüche 1 bis 3,
**dadurch gekennzeichnet,**
**dass** die Verzwirbelung als halber Schlag ausgebildet ist, wobei der halbe Schlag einer Umschlingung des Drahtes von 180° entspricht.

5. Komprimierbarer, selbstexpandierender Stent nach einem der Ansprüche 1 bis 4,
**dadurch gekennzeichnet,**
**dass** im nicht expandierten Zustand des Stents der Abschnitt des Drahtes, der zu einer Verzwirbelung umschlungen ist, derart aufgeweitet ist, dass im Bereich der Verzwirbelung ein elliptisch aufgeweiteter Abschnitt ausgebildet ist.

6. Komprimierbarer, selbstexpandierender Stent nach Anspruch 5,
**dadurch gekennzeichnet,**
**dass** im elliptisch aufgeweiteten Abschnitt des Drahtes zwei Reibungsabschnitte ausgebildet sind, in denen sich jeweils zwei Abschnitte des Drahtes kontaktieren.

7. Komprimierbarer, selbstexpandierender Stent nach einem der Ansprüche 1 bis 6,
**dadurch gekennzeichnet,**
**dass** der Übergangsabschnitt konisch aufweitend ausgebildet ist.

8. Komprimierbarer, selbstexpandierender Stent nach einem der Ansprüche 1 bis 7,
**dadurch gekennzeichnet,**
**dass** das Geflecht aus sich kreuzenden Strängen ausgebildet ist.

9. Komprimierbarer, selbstexpandierender Stent nach einem der Ansprüche 1 bis 8,
**dadurch gekennzeichnet,**
**dass** die Verzwirbelung zweier Abschnitte als mindestens ein ganzer Schlag ausgebildet ist, wobei der ganze Schlag einer Umschlingung der zwei Abschnitte des Drahtes von 360° entspricht.

10. Komprimierbarer, selbstexpandierender Stent nach einem der Ansprüche 1 bis 9,
**dadurch gekennzeichnet,**
**dass** das Geflecht eine Vielzahl von Öffnungen aufweist, wobei die Öffnungen im Übergangsabschnitt größer sind als die übrigen Öffnungen des proximalen Abschnitts, wodurch der Übergangsabschnitt in expandiertem gebogenen Zustand eine aufgeweitete Form einnimmt.

11. Vorrichtung zur Schienung und/oder Offenhaltung des Rachenraums mit zumindest einem in einem Einführschlauch komprimierbaren und selbstexpandierenden Stent, wobei der Stent ein Stent nach einem der Ansprüche 1 bis 10 ist.

12. Vorrichtung nach Anspruch 11,
**dadurch gekennzeichnet,**
**dass** ein Abschnitt des Einführschlauches kreissegmentförmig gebogen ist, um das Einführen durch einen Nasengang in den Rachenraum zu erleichtern, wobei der Abschnitt eine Biegung von etwa 80° bis 120° aufweist.

## Claims

1. Compressible self-expanding stent for splinting and/or keeping open a cavity, an organ duct and/or a vessel in the human or animal body, whereas the stent is formed of at least one wire and comprises a distal section, a proximal section and a transition section formed therebetween, whereas the distal section has a greater expansion than the proximal section, and these sections comprise a braid, and wherein sections of the wire or wires extending between a proximal and a distal end of the stent are referred to as strands, wherein in the region of the proximal section adjacent to the transition section in the proximal direction, a decoupling region is formed in which at least two strands are twisted together, **characterized in that** said strands extend approximately in the longitudinal direction of the stent.

2. Compressible, self-expanding stent according to claim 1,
**characterized in that**
the strands which run in the decoupling region approximately in the longitudinal direction of the stent form the distal section with a constant diameter in such a way that it is approximately tubular.

3. Compressible, self-expanding stent according to claim 1 or 2,
**characterized in that**
at the distal end of the stent two strands are connected via a connecting section and each connecting section is bent to form an loop, wherein each loop is forming a twist by enlacing of the each two strands with which the loop is closed.

4. Compressible self-expanding stent according to any one of claims 1 to 3,
**characterized in that**
the twist is formed as a half-twist, wherein the half-twist is corresponding to a 180° enlacement of the wire.

5. Compressible self-expanding stent according to any one of claims 1 to 4,
**characterized in that**
in the unexpanded state of the stent the section of the wire that is enlaced to form a twist is expanded such that an elliptically widened section is formed in the region of the twist.

6. Compressible self-expanding stent according to claim 5,
**characterized in that**
in the elliptically widened section of the wire two friction sections are formed, in which each two sections of the wire contact each other.

7. Compressible self-expanding stent according to any one of claims 1 to 6,
**characterized in that**
the transition section is provided conically widening.

8. Compressible self-expanding stent according to any one of claims 1 to 7,
**characterized in that**
the mesh is formed by intersecting strands.

9. Compressible self-expanding stent according to any one of claims 1 to 8,
**characterized in that**
the twisting of two sections is formed as at least one full stitch, whereas the full stitch corresponds to a 360° enlacement of the two sections of the wire.

10. Compressible self-expanding stent according to any one of claims 1 to 9,
**characterized in that**
the mesh has a plurality of openings, whereas the openings in the transition section are larger than the other openings of the proximal section, so that the transition section assumes a widened shape in the expanded bent state.

11. Apparatus for splinting and/or keeping open the pharynx comprising at least one self-expanding stent compressible in an insertion tube, whereas the stent is a stent according to any one of claims 1 to 10.

12. Apparatus according to claim 11,
**characterized in that**
a section of the insertion tube is bent in a circular segment shape to facilitate insertion through a nasal passage into the pharynx, whereas the section has a bend of about 80° to 120°.

## Revendications

1. Endoprothèse compressible et auto-expansible permettant de soutenir et/ou de maintenir ouverte une cavité, un conduit organique et/ou un vaisseau dans le corps humain ou animal, l'endoprothèse étant formée d'au moins un fil et comprenant une section distale, une section proximale et une section de transition formée entre les deux, la section distale présentant un élargissement plus grand que celui de la section proximale et les sections présentant une tresse, et des sections du fil ou des fils, lesquelles s'étendent entre une extrémité proximale et une extrémité distale de l'endoprothèse, sont appelés brins, une zone de découplage étant formée dans la zone de la section proximale adjacente à la section de transition dans la direction proximale, dans laquelle zone de découplage au moins deux brins sont torsadés ensemble, **caractérisée en ce que** lesdits brins s'étendent approximativement dans la direction longitudinale de l'endoprothèse.

2. Endoprothèse compressible et auto-expansible selon la revendication 1, **caractérisée en ce que** les brins qui s'étendent approximativement dans la direction longitudinale de l'endoprothèse dans la zone de découplage forment la section distale avec un diamètre constant de telle sorte que ladite section distale est approximativement tubulaire.

3. Endoprothèse compressible et auto-expansible selon la revendication 1 ou 2, **caractérisée en ce qu'**à l'extrémité distale de l'endoprothèse, deux brins sont reliés par l'intermédiaire d'une section de liaison et chaque section de liaison est coudée pour former un oeillet, chaque œillet formant une torsade en s'enroulant autour des deux brins respectifs, laquelle torsade ferme l'œillet.

4. Endoprothèse compressible et auto-expansible selon l'une des revendications 1 à 3, **caractérisée en ce que** la torsade est réalisée sous forme de demi-tour, le demi-tour correspondant à un enroulement du fil à 180°.

5. Endoprothèse compressible et auto-expansible selon l'une des revendications 1 à 4, **caractérisée en ce qu'**à l'état non expansé de l'endoprothèse, la section du fil qui est enroulée pour former une torsade est élargie de telle sorte qu'une section élargie elliptiquement est formée dans la zone de la torsade.

6. Endoprothèse compressible et auto-expansible selon la revendication 5, **caractérisée en ce que** deux sections de friction sont formées dans la section élargie elliptiquement du fil, dans chacune desquelles respectivement deux sections du fil sont en contact.

7. Endoprothèse compressible et auto-expansible selon l'une des revendications 1 à 6, **caractérisée en ce que** la section de transition est formée de façon à s'élargir de manière conique.

8. Endoprothèse compressible et auto-expansible selon l'une des revendications 1 à 7, **caractérisée en ce que** la tresse est formée de brins croisés.

9. Endoprothèse compressible et auto-expansible selon l'une des revendications 1 à 8, **caractérisée en ce que** la torsade de deux sections est réalisée en au moins un tour complet, le tour complet correspondant à un enroulement à 360° des deux sections du fil.

10. Endoprothèse compressible et auto-expansible selon l'une des revendications 1 à 9, **caractérisée en ce que** la tresse présente une pluralité d'ouvertures, les ouvertures dans la section de transition étant plus grandes que les ouvertures restantes de la section proximale, de sorte que la section de transition prend une forme élargie à l'état expansé et courbé.

11. Dispositif permettant de soutenir et/ou de maintenir le pharynx ouvert, comportant au moins une endoprothèse compressible et auto-expansible dans un tube d'insertion, l'endoprothèse étant une endoprothèse selon l'une des revendications 1 à 10.

12. Dispositif selon la revendication 11, **caractérisé en ce qu'**une section du tube d'insertion est courbée en segment de cercle pour faciliter son insertion dans le pharynx par la voie nasale, la section présentant une courbure d'environ 80 à 120°.
